# EUROPEAN PATENT APPLICATION

(11) **EP 0 990 427 A2**
(43) Date of publication of application: **05.04.2000**
(21) Application number: 99118790.7
(22) Date of filing: 23.09.1999
(51) Int. Cl.: A61F 2/06

(54) **Systems and methods for mounting a stent on a catheter**

(30) Priority: 30.09.1998 US 163634
(71) Applicant: Medtronic AVE, Inc., Santa Rosa, California 95403 (US)
(72) Inventor: Fagan, John R., Pepperell, MA 01463 (US); Gilbert, Joanne, Amesbury, MA 01913 (US); Moore, Timothy P., Newton, MA 02466 (US); Morrill, Richerd J., Billerica, MA 01821 (US); Choc, Richard T., Waltham, MA 02154 (US)
(74) Representative: Bauer, Friedrich, Dipl.-Ing.

(57) **Abstract**

The invention provides systems and methods for stent mounting devices that have a radially expandable sleeve and a movable collar. The sleeve and the movable collar can mount about the distal end of a catheter in an arrangement that allows the movable collar to slide over the catheter to push against the sleeve. A stent can fit over and sit around the periphery of the sleeve. Responsive to the pushing of the collar, the sleeve can radially expand from a first diameter to a second, larger diameter, thereby creating an interference fit between the sleeve and the stent, to hold the stent more tightly to the catheter delivery system. The device can be employed with self-expanding or balloon expandable stents.

## Description

### Field of the Invention

The invention relates to systems and methods for mounting a stent onto a catheter, and more particularly, to systems and methods for maintaining a stent onto a catheter as the catheter travels through a body lumen to the site of treatment.

### Background of the Invention

Diseases of the vascular system, including cardiac artery disease, afflict a substantial portion of the adult population. Occlusion of cardiac arteries being one of the more common diseases. In addressing these diseases, surgeons have developed a number of surgical procedures that provide effective treatment. However, the surgeries themselves are often highly invasive and can be taxing and dangerous for the patient.

To provide less taxing treatments, surgeons have developed percutaneous transluminal angioplasty (PTA) and percutaneous transluminal coronary angioplasty (PTCA) techniques that reduce arterial build-up of cholesterol fats or atherosclerotic plaque. In a typical PTA or PTCA procedure, a balloon catheter is advanced through a guide catheter so that the balloon of the balloon catheter is properly positioned within the stenosed region of the blood vessel. To alleviate the deleterious effects of the stenosis, the balloon at the distal end of the catheter is inflated to widen the blood vessel at the site of the stenosis. These techniques are generally quite successful in achieving an initial widening of an occluded vessel, however in thirty to fifty percent of cases, the initial increase in lumen dimensions is followed by a localized re-narrowing (restenosis) of the vessel over a period of three to six months. Gibbons *et at, Molecular Therapies for Vascular Diseases*, Science vol. 272, pages 617-780 (May 1996). To prevent reclosure or restenosis, a metal stent is typically deployed during the PTA or PTCA procedure to reinforce the blood vessel wall and to hold any flaps, fissures and dissections in place. A typical stent comprises a tubular structure that expands radially from a compact form for transit to an expanded form for implantation. Radial expansion causes the stent to implant into the tissue of a wall of the vessel being repaired or bridged to maintain its patency.

To place the stent at the desired site, the surgeon employs a stent delivery system that typically comprise an elongate catheter body having a transverse dimension that allows the delivery system to travel through a patient's lumens and to the desired site. Most of the existing delivery systems are provided separately from the stent which is to be placed within the patients vessel. Accordingly, the surgeon is required to mount the stent to the distal end of the delivery system. This involves the surgeon taking the stent in hand and sliding it over the distal end of the elongate catheter body. In the case of a self-expanding stent, the surgeon slides the stent directly over the catheter body, and in the case of a balloon expandable stent, the surgeon slides the stent over a wrapped balloon that is located at the distal end of the stent delivery catheter.

Once positioned onto the catheter, the surgeon typically pinches, or squeezes, the stent gently in order that the stent engage frictionally with the surface of the balloon. This is known as crimping. The point of crimping is to form the stent into an engagement with the catheter, or balloon mounted on the catheter, to cause the stent to fit snugly onto the catheter and fill any and all gaps that may exist between the stent in its predeployed condition and the catheter. It is understood that by crimping the stent onto the balloon, the surgeon minimizes the opportunity for the stent to slide off the balloon, or distal end of the catheter, as the stent is delivered through the lumen of the patient.

Generally, the crimping process is deemed unsatisfactory. First, it requires the surgeon to actually physically deform the stent, which, although typically quite acceptable, still runs the risk of misshaping the stent to a point that may interfere with the ability of the stent to expand exactly as the surgeon had anticipated. Moreover, the frictional engagement that arises from crimping the stent onto the catheter may be insufficient to guarantee that the stent will maintain its position during delivery as the stent is guided through the body lumen.

To improve the deliverability of stents during these processes a number of existing systems have been proposed, including systems that provide sheaths that can be slid over the stent once it is mounted onto the catheter. For these systems, the surgeon mounts the stent onto the catheter, slides a sheath over the stent, to fix the stent onto the distal end of the catheter, and then once the stent is positioned within the body lumen, pulls on a proximal end of the sheath to slide the sheath off the stent thereby allowing the stent to be deployed. Although these systems can work well, they require that an additional step be taken prior to the surgeon continuing with the procedure, that step being applying a sheath to the stent. Moreover, it is not uncommon that during the step of sliding the sheath off the stent, the stent is actually moved as well, thereby causing the surgeon to have to reposition the stent within the body lumen. Accordingly, the sheath solution, although often working effectively, does require extra steps by the surgeon, and can itself lead to unwanted sliding of the stent prior to deployment. A further solution to this problem has been proposed wherein dams have been formed onto the delivery catheter such that proximal and distal dams act as mechanical stops so that a stent placed between the two dams cannot slide proximally or distally as the dams prevent unwanted lateral movement of the stent. One such system is shown is U.S. Patent 5,632,760. Although these systems can work well, the dams have the unfortunate effect of increasing the transverse dimension of the stent delivery system. As discussed above, it is almost always more desirable to have as small a diameter delivery system as possible, given that the delivery system is to be pushed and guided through the patients body lumen to effect delivery.

Accordingly, there is a need in the art for improved delivery systems that have greater ease of use, and yet do not give rise to unwanted increases in transverse dimensions of delivery systems.

### Summary of the Invention

It is an object of the invention to provide improved systems and methods for mounting a stern to a catheter.

Other objects of the invention will, in part, be obvious, and, in part, be shown from the following description of the systems and methods shown herein.

The systems and methods described herein include stent mounting devices that have a radially expandable sleeve and a movable collar. A stent can fit over and sit around the periphery of the sleeve. The sleeve and collar can mount about the distal end of a catheter in an arrangement that allows the movable collar to slide over the catheter to push against the sleeve. Responsive to the pushing of the collar, the sleeve can radially expand from a first diameter to a second, larger diameter, thereby creating an interference fit between the sleeve and the stent, to hold the stent more tightly to the catheter delivery system. The device can be employed with self-expanding or mechanically expandable stents.

More particularly, the systems and methods described herein include apparatus for maintaining a stent on a catheter, comprising a sleeve formed of a longitudinally compressible material and disposed about the distal end of a catheter, the sleeve being radially expandable from a first diameter to a larger second diameter, and a collar positioned adjacent the sleeve and disposed about the exterior of the catheter, whereby moving the collar inwardly against the sleeve actuates the sleeve to expand from the first diameter to the larger second diameter. In alternative embodiments, the sleeve can comprise a tube of resilient material, a set of bellows, including bellows having a pitch selected to create an interlocking engagement with a set of gaps in a wall of the stent, and a helical body wrapped around a portion of the catheter, including a helical body wrapped around a portion of the catheter and having a pitch selected to cause an interlocking engagement with a set of gaps in a wall of the stent.

The collar can comprises a ring of resilient material and the sleeve and collar can be dimensionally adapted for being disposed sitting beneath the balloon of the balloon catheter.

In another aspect, the system comprises a stent delivery system including a catheter, having an elongate catheter body having a proximal end and a distal end, a sleeve formed of a longitudinally compressible material and disposed about the distal end of the catheter body, the sleeve being radially expandable from a first diameter to a larger second diameter, a collar positioned adjacent the sleeve and disposed about the exterior of the catheter body and optionally, a balloon can be mounted at the distal end of the catheter body and fit over the sleeve and the collar.

The sleeve can comprise a tube of resilient material, a bellows and or a helical body wrapped around a portion of the catheter. In another aspect the invention includes methods for mounting a stent to a balloon of a balloon catheter, comprising the acts of providing a balloon catheter having an elongate catheter body with a balloon mounted on the catheter body, wherein the balloon is mounted over a sleeve formed of a longitudinally compressible material, and a collar positioned adjacent the sleeve and disposed about the exterior of the catheter body, fitting a stent over the balloon, and moving the collar against the sleeve to expand the sleeve from a first diameter to a larger second diameter, whereby the sleeve drives the balloon into frictional engagement with the stent. Similar methods are provided for mounting a self-expanding stent to a catheter of a catheter delivery system.

In a further aspect the invention can be understood as a method of manufacturing a catheter comprising the acts of providing an elongate catheter body having a proximal end and a distal end, disposing a sleeve formed of a longitudinally compressible material about the distal end of the catheter body, the sleeve being radially expandable from a first diameter to a larger second diameter, disposing a collar adjacent the sleeve and about the exterior of the catheter body, and mounting a balloon mounted on the distal end of the catheter body and over the sleeve and the collar.

### Brief Description of the Drawings

The foregoing and other objects and advantages of the invention will be appreciated more fully from the following further description thereof, with reference to the accompanying drawings wherein;.
Figure 1 depicts one embodiment of a stent mounting system for forming an interference fit with a stent;
Figure 2 depicts an embodiment of a stent mounting system for forming an interference fit with a stent;
Figure 3 depicts one embodiment of a stent mounting system for forming an interference fit with a stent;
Figure 4 depicts one embodiment of a stent mounting system for forming an interference fit with a stent; and
Figure 5 depicts one embodiment of a stent mounting system for forming an interference fit with a stent.

### Detailed Description of the Illustrated Embodiments

To provide an overall understanding of the invention, certain illustrative embodiments will now be described, including a system for mounting a stent onto the balloon of a balloon catheter. However, it will be understood by one of ordinary skill in the art that the systems described herein can be adapted and modified to provide systems that can mount self-expanding stents to a catheter, as well as for mounting stent-grafts or for any other suitable prosthetic. Other additions and modifications can be made to the invention without departing from the scope hereof.

The systems and methods described herein include stent mounting devices that allow for a stent to be mounted onto a catheter deliver system that will deliver the stent to the site of treatment within the patient. The systems and methods described herein can be employed with self-expanding stents as well as mechanically expandable stents, such as balloon stents. As will be seen from the following illustrative embodiments, it will be understood that the systems of the invention include stent mounting systems that have a sleeve of resilient material that slides over the catheter body and that can be disposed at a distal end of the catheter. A collar made of resilient material can also be slid onto the catheter body and pushed up adjacent to the sleeve. Once the sleeve and collar are arranged onto the catheter body, a balloon can optionally be secured to the catheter body in a manner that sits the sleeve and collar within the interior of the balloon. In those embodiments that include self-expanding stents, no balloon is present.

In operation, a surgeon can slide the stent over the sleeve and then move the collar into the sleeve to cause the sleeve to expand from a first diameter to a second, larger diameter. In the radially expanded condition, it is understood that the sleeve diameter is sufficiently large to form a frictional interference engagement with the stent, thereby affixing the stent more securely to the catheter body. It is understood that stents so mounted are less likely to slide off the catheter during delivery through the patient's vasculature, or other body lumen.

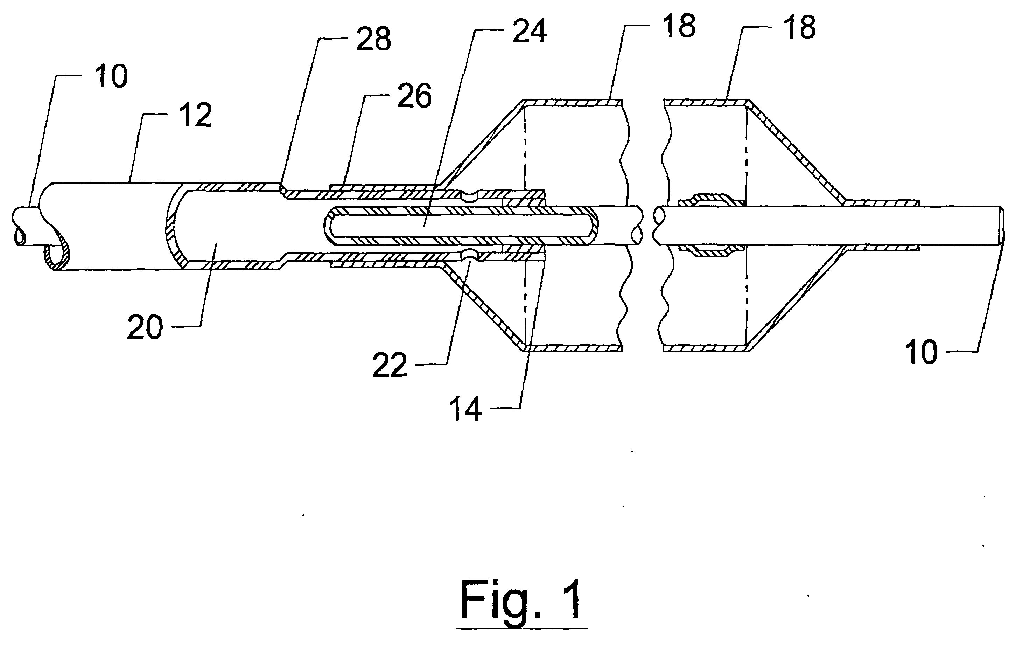

More particularly, the systems described herein include the system 10 depicted in Figure 1. Specifically, Figure 1 shows a stent mounting system 10 that includes a sleeve 12, a collar 14, and an elongate catheter body 16, a balloon 18, and a stent 20. Figure 1 depicts the stent mounting system in its expanded condition, that is in a condition wherein the collar 14 has been slid into the sleeve 12 to cause the sleeve 12 to radially expand and engage the stent 20. For purposes of reference hereinafter, it will be understood that the collar 14 is adjacent the distal end of the sleeve 12.

The depicted embodiment is a stent mounting system for a balloon expandable stent. As shown by Figure 1, the collar 14 and sleeve 12 are dimensionally adapted such that both elements 12 and 14 can be disposed within the interior space defined by the balloon 18 (shown in Figure 1 in a rapt configuration). The stent 20 is shown as being mounted over the balloon 18 and the sleeve 12. The stent 20 can be manually crimped onto the catheter 16 by the manual manipulation of the surgeon as is conventionally done when securing a stent to the body of a catheter delivery system. Optionally, the crimping step can be eliminated or minimized by further compression of the sleeve 12 by movement of the collar 14, to thereby achieve greater radial expansion of the sleeve 12.

The depicted sleeve 12, shown in cutaway in Figure 1, can be formed as a cylindrical sleeve of resilient material that can be fitted and slid over the elongate catheter body 16. Alternatively, the sleeve 12 can be formed from a plurality of strips of resilient material that are mounted to the catheter 16 to extend laterally along a portion of the catheter, particularly that portion to which the stent 20 will be mounted. In one embodiment, the sleeve 12 is secured to the catheter body 16 by affixing the proximal end of the sleeve 12 to the catheter body 16. For example, the proximal end of the sleeve 12 can be epoxied to the catheter body 16 at the proximal end of the sleeve, thereby preventing the sleeve 12 from moving along the surface of the catheter 16. Moreover, by affixing the proximal end, the distal end is left free to be compressed by the action of the collar 14, thereby facilitating the radial expansion of the sleeve 12.

The depicted sleeve 12 can be formed of any resiliant material including plastic, rubber or foam, as well as natural materials such as cotton. However, different materials can be employed for forming the sleeve 12 without departing from the scope of the invention.

The depicted collar 14 is shown as a short collar that has a central aperture that can receive the elongate catheter body 16. The collar 14 is adapted to butt against the side of the sleeve 12, in order that longitudinal movement of the collar 14 will cause the compression and radial expansion of the sleeve 12. The collar 14 can be made of any suitable material including rubber, plastic or foam. The collar is designed to fit tightly about the catheter, so that the collar 14 stays in place once positioned by the surgeon. However, it will be understood by those of ordinary skill in the art that any suitable material can be employed for forming the collar 14 and any such modifications or substitutions are within the scope of the invention.

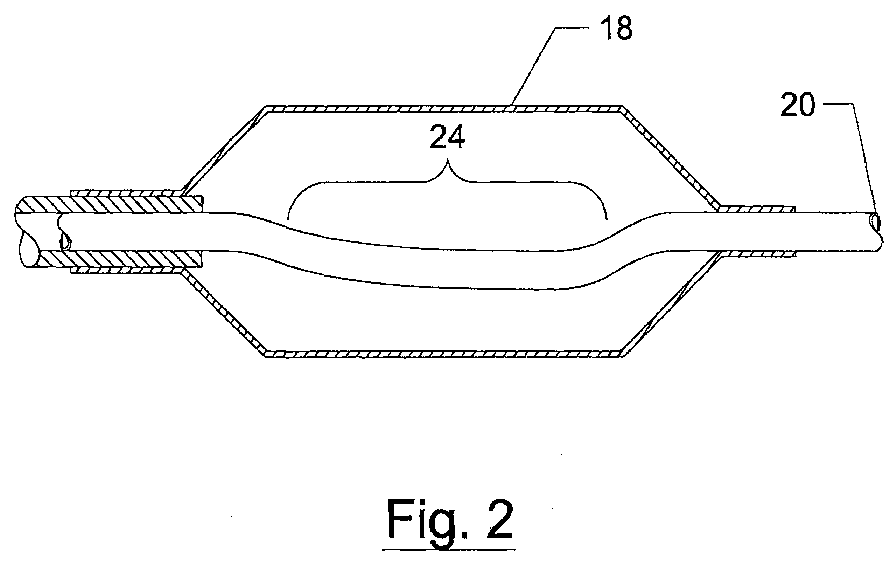

Figure 2 depicts the stent mounting system 10 of Figure 1 in a condition wherein the balloon 18 is expanded. Specifically, Figure 2 shows the stent 20 in an expanded configuration that is achieved by inflating balloon 18 to drive the stent 20 off the sleeve 12, and to expand the stent 20 for deployment within a body lumen such as a cardiac artery. Figure 2 depicts the collar 14 as having moved longitudinally and away from the sleeve 12. Movement of the collar 14 can occur on expansion of the balloon 18, however, it is understood that balloon 18 is not expanded until the stent 20 is placed at the location to be deployed and patency to be maintained. In another embodiment, the collar 14 can be fitted sufficiently tightly over the catheter 16 such that expansion of the balloon 18 will not cause any movement of the collar 14 either distally or proximately.0

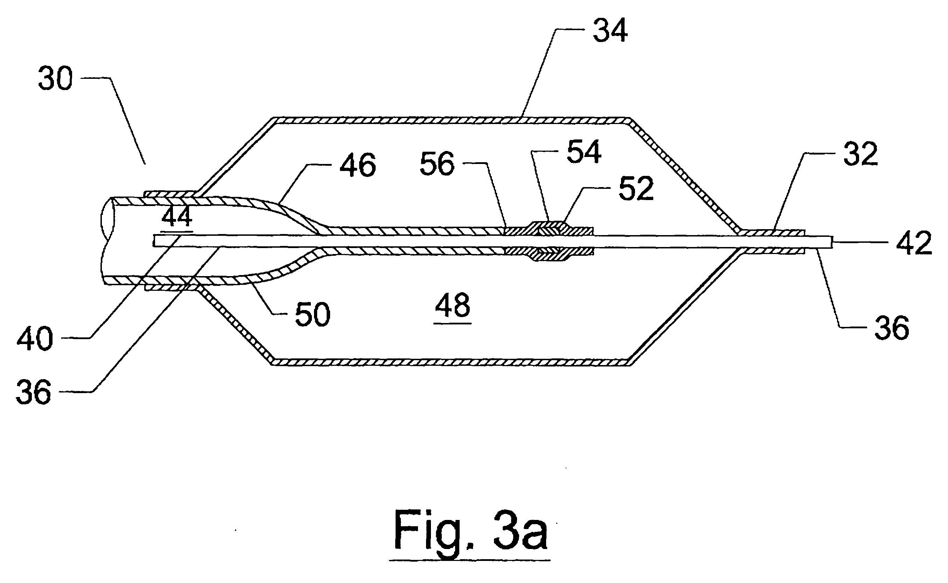

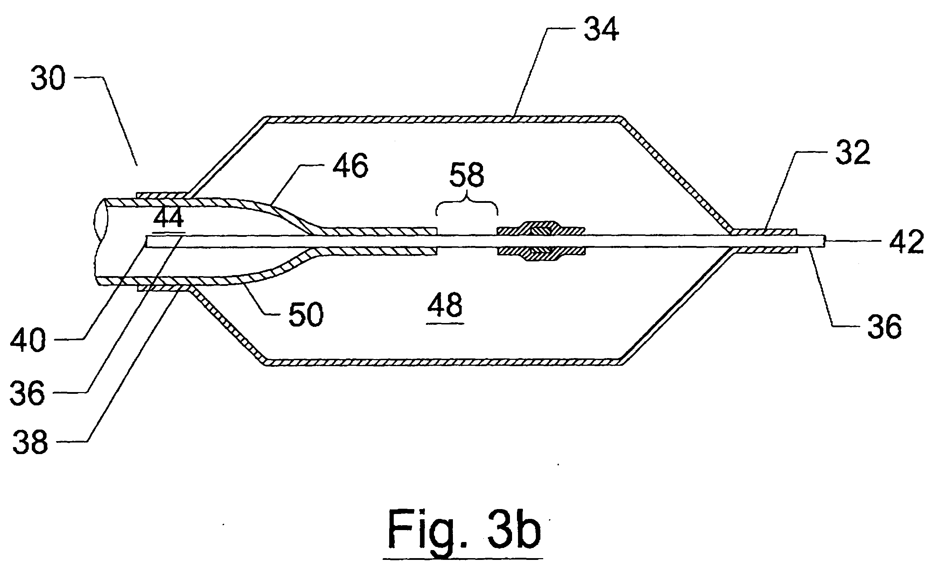

Figure 3 depicts a further system according to the invention. Specifically, Figure 3 depicts a system 30 that includes a sleeve 32 formed as a helically wrapped body that is disposed about the periphery of catheter 38. In the embodiment-depicted, the catheter 38 is a catheter for delivering a balloon expandable stent that can be opened by action of the depicted balloon 40.

In the embodiment depicted in Figure 3, the helically wrapped sleeve 32 is fixed to the catheter 38 at a proximal location (not shown). In operation, sliding the collar 34 proximally causes the collar 34 to butt against and compress the helically wrapped sleeve 32; resulting in compression and radial expansion thereof. For the embodiment depicted in Figure 1 the radially expanding helically body can result in portions of the helical body fitting into space that occur in the side wall of the stent. Accordingly, in one practice the helical sleeve can have coils that are spaced at a pitch that is compatible with the pitch between spaces in the well of the stent 36. In this way, compression of the sleeve 32 can result in an interference fit with the spaces that occur in the wall of the stent 36.

The depicted helical sleeve 32 can be formed of a biocompatible material that provides a spring like body that has a tendency to unwind and therefore expand radially as the coils are compressed together. In other embodiments the helical sleeve 32 can be formed of other materials, and it will be understood to one of ordinary skill of the art, that any material suitable for forming the helical sleeve 32 depicted in Figure 3 can be employed by the invention without departing from the scope thereof.

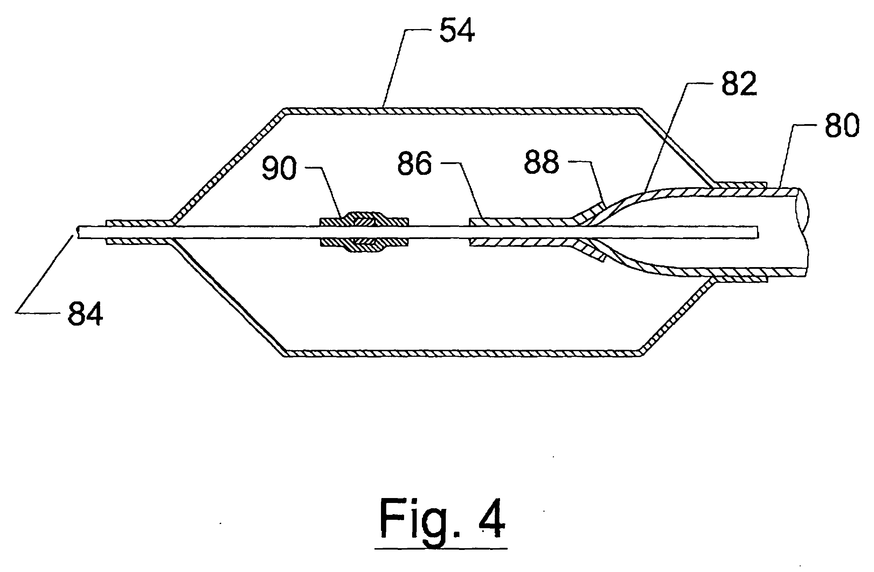

Figure 4 depicts a further alternative embodiment of a stent mounting system according to the invention. Specifically, Figure 4 depicts a stent mounting system 40 that includes a set of bellows 42 that act as a sleeve which fits around a portion of the catheter 44. Along with the sleeve 42 is a collar 48 that sits around the periphery of the catheter 44 and is disposed adjacent the bellows 42. As further shown by Figure 4, the system 40 is adapted for providing a stent mounting system that can work with a balloon expandable stent such as the stent 50 that is expandable by action of the balloon 52. As discussed above with reference to Figure 3, the stent 50 has an exterior wall that includes a number of spaces which are longitudinally spaced apart along the wall. The bellows 52 has a series of peaks such as peak 54 that, upon compression of the bellows 42, will regularly expand, and extend into the spaces 58 that occur in the stent wall 50. In this way, the compression of bellows 42 results in an interference fit with the spaces 58 in the stent 50 and provides for greater securing of the stent 50 onto the catheter 44 of the catheter delivery system.

The bellows 42 depicted in Figure 4 can be formed of any suitable material including rubber or plastic and will be dimensionally adapted such that the compression of the bellows 42 results in the radial expansion of the bellows 42 from a first diameter to a second larger diameter, wherein that second larger diameter is sufficiently large to engage the stent 50, thereby creating either frictional engagement or an interference fit with the stent 50. The depicted collar 48 can be similar to any of the collars with reference Figures 1 through 3.

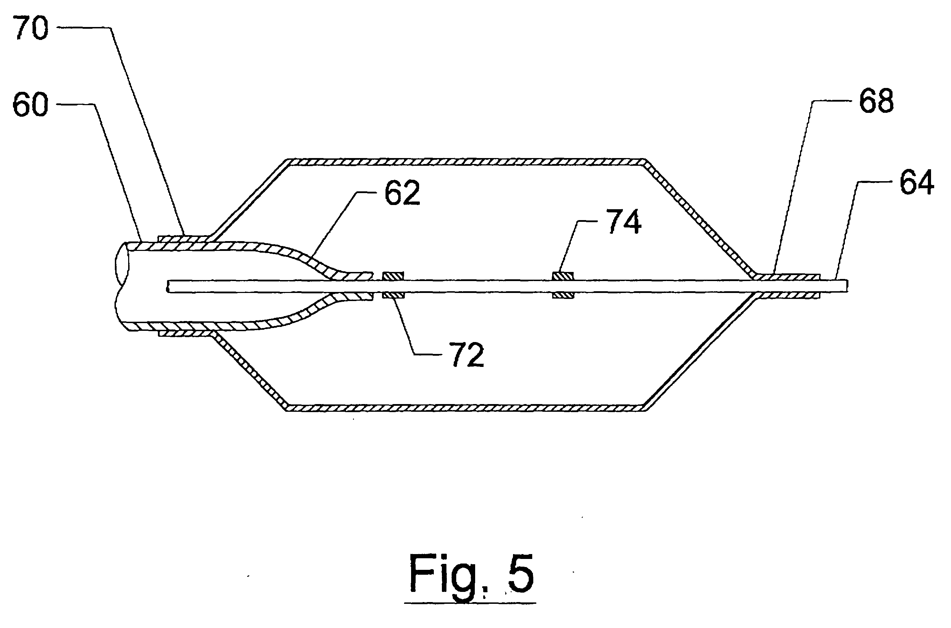

Figure 5 depicts a further alternative embodiment of the invention wherein the depicted system 60 provides a stent mounting system for a catheter 62. Specifically, the stent mounting system shown in Figure 5 is dimensionally adapted to fit underneath the balloon 64 and includes a collar 66 and a helically wrapped sleeve 70. The helically wrapped sleeve 70 has a pitch between coils that is selected to create an interference fit upon the compression of the helically wrapped sleeve 70. Specifically, as depicted by Figure 5 upon compression of the helical sleeve 70 by movement of the collar 66, the helical sleeve 70 can expand from first diameter to a second larger diameter. Moreover, upon compression, the coils can be aligned with the spaces 72 that occur within the wall of the stent 68. In this way, the stent 68 is held by an interference fit that is created by the coils of the helical sleeve 70 and can extend into the spaces in 72 that occur within the wall of the stent 68.

Although the above illustrated embodiments depict mechanically expandable stents that are driven open by action of an expanding balloon, it will be understood that the systems and methods described herein can be employed with any type of mechanically expandable stent or endoprostheses, including other types of mechanically expandable stents as well as self expanding stents, such as stents made of nickel-titanium compositions. Similarly, the systems and methods of the invention are not limited to use with any of the delivery systems depicted or described above, and can be employed with any delivery system such as a catheter delivery system that includes a catheter with associated balloon inflation, or stent recapture apparatus. Such delivery systems allow the surgeon to guide the distal end carrying the stent to a selected location in the patient's body. The surgeon can then operate the control apparatus to release the stent at the selected location, or recapture the stent if so desired. It will also be understood that the systems described herein can include a plurality of collars, located both distally and proximally of the compressibly expandable sleeve. The systems described herein can also be employed with other stent holding devices, including sheaths and dams, as well as with mechanisms that can cause the radially expansion of the sleeve, such as mechanisms that can be controlled by the surgeon during delivery of the stent to create a radially expansive force that engages the stent and holds the stent to the delivery system.

Those skilled in the art will know or be able to ascertain using no more than routine experimentation, many equivalents to the embodiments and practices described herein. For example, the systems and methods described herein can be employed with stents being disposed in body canals, blood vessels, ducts and other body passages. Additionally, both the collar and sleeve can have shapes other than those shown in the depicted embodiments, and can be made of any suitable materials. Further, other embodiments can be realized, wherein the collar and the sleeve are integrally formed as a single component that can be mounted onto the catheter. Accordingly, it will be understood that the invention can be realized by many different systems that include a radially expandable sleeve and is not to be limited to the embodiments disclosed herein, but is to be understood from the following claims, which are to be interpreted as broadly as allowed under the law.

## Claims

1. Apparatus for maintaining a stent on a catheter, comprising
a sleeve formed of a longitudinally compressible material and disposed about the distal end of a catheter, said sleeve being radially expandable from a first diameter to a larger second diameter, and
a collar positioned adjacent said sleeve and disposed about the exterior of the catheter, whereby moving said collar against said sleeve actuates said sleeve to expand from said first diameter to said larger second diameter.

2. Apparatus according to claim 1, wherein said sleeve comprises a tube of resilient material.

3. Apparatus according to claim 1, wherein said sleeve comprises bellows.

4. Apparatus according to claim 1, wherein said sleeve comprises bellows having a pitch selected to create an interlocking engagement with a set of gaps in a wall of the stent.

5. Apparatus according to claim 1, wherein said sleeve comprises a helical body wrapped around a portion of said catheter.

6. Apparatus according to claim 1, wherein said sleeve comprises a helical body wrapped around a portion of said catheter and having a pitch selected to cause an interlocking engagement with a set of gaps in a wall of the stent.

7. Apparatus according to claim 1, wherein said collar comprises a ring of resilient material.

8. Apparatus according to claim 1, wherein said sleeve and collar are dimensionally adapted for being disposed beneath the balloon of a balloon catheter.

9. A balloon catheter, comprising
an elongate catheter body having a proximal end and a distal end,
a sleeve formed of a longitudinally compressible material and disposed about the distal end of the catheter body, said sleeve being radially expandable from a first diameter to a larger second diameter,
a collar positioned adjacent said sleeve and disposed about the exterior of the catheter body, and
a balloon mounted at said distal end of said catheter body and fit over said sleeve and said collar.

10. A balloon catheter according to claim 9, wherein said sleeve comprises a tube of resilient material.

11. A balloon catheter according to claim 9, wherein said sleeve comprises a bellows.

12. A balloon catheter according to claim 9, wherein said sleeve comprises a helical body wrapped around a portion of said catheter.

13. A method for mounting a stent to a catheter, comprising
providing a balloon catheter having an elongate catheter body with a balloon mounted on said catheter body, wherein said balloon is mounted over a sleeve formed of a radially expandable material, and a collar positioned adjacent said sleeve and disposed about the exterior of the catheter body,
fitting a stent over said balloon, and
moving said collar against said sleeve to expand said sleeve from a first diameter to a larger second diameter, whereby said sleeve drives said balloon into frictional engagement with said stent.

14. A method of manufacturing a catheter, comprising,
providing an elongate catheter body having a proximal end and a distal end,
disposing a sleeve formed of a longitudinally compressible material about the distal end of the catheter body, said sleeve being radially expandable from a first diameter to a larger second diameter, and
disposing a collar adjacent said sleeve and about the exterior of the catheter body.

15. A method according to claim 14, further comprising
mounting a balloon on said distal end of said catheter body and over said sleeve and said collar.

16. A method according to claim 14, wherein said act of disposing a sleeve includes the act of providing a sleeve comprising a tubular resilient body.

17. A method according to claim 14, wherein said act of disposing a sleeve includes the act of providing a sleeve comprising bellows.

18. A method according to claim 14, wherein said act of disposing a sleeve includes the act of providing a sleeve comprising a helical body.
